# EUROPEAN PATENT APPLICATION

(11) **EP 1 197 269 A2**
(43) Date of publication of application: **17.04.2002**
(21) Application number: 02000396.8
(22) Date of filing: 29.09.1995
(51) Int. Cl.: B08B 3/02, B08B 3/00, C02F 3/00

(54) **Microbiological parts washer**

(30) Priority: 30.09.1994 US 315902; 10.01.1995 US 370898
(62) Divisional of application: 95936304.5
(71) Applicant: ZYMO International Inc., Duluth, GA 30096 (US); CHEMFREE CORPORATION, Norcross, GA 30093 (US)
(72) Inventor: McClure, James C., Norcross, GA 30092 (US); Mears, Eric L., Duluth, GA 30155 (US); Whiteman, G. Rob, Duluth, GA 30136 (US); Marks, Frank A, Atlanta, GA 30327-4327 (US); Mc Nally, Thomas W, Norcross, GA 30092-1409 (US); Leland Strange, J, Duluth, Ga (US)
(74) Representative: Grund, Martin, Dr.

(57) **Abstract**

An apparatus includes a first mechanical component for cleaning parts by contacting the parts with a fluid, and a second mechanical component for biodegrading the organic matter removed from the parts. The first mechanical component is in communication with the second mechanical component so that the fluid recirculates between the first and second mechanical components. A method of washing parts including the steps of placing an article in a first chamber (20), circulating a washing liquid (82) from a second reservoir (80) to the first reservoir (20) to wash the surfaces of the article in contact with the liquid, passing the washing liquid (82) through a porous medium (38), draining the washing liquid from the first chamber (20) into the second chamber (80), removing organic matter in the washing liquid (82) and recirculating the washing liquid from the second chamber (80) to the first chamber (20), is also provided.

## Description

### BACKGROUND OF THE INVENTION

The present invention relates generally to an apparatus and process for washing parts, and more particularly, to an apparatus and process for washing parts using bioremediation.

Parts washers have been developed to clean objects contaminated with organic waste products. These conventional parts washers traditionally use chlorinated solvents, mineral spirits solvents, other organic solvents, aqueous detergents or surfactant blends for cleaning fluids. Although these cleaning fluids may be effective for cleaning parts, there are many drawbacks to their use in parts washing. In particular, chlorinated solvents, mineral spirits solvents, and terpene based solvents are presently classified by government regulatory agencies as hazardous materials because of their low flash point and potential health concerns. Because of this classification, these solvents must be used, handled, and disposed of in compliance with extensive government regulations. For example, the disposal of mineral spirits solvents can be expensive and is usually achieved at special hazardous waste recycling plants. Furthermore, mineral spirits solvents that are not properly contained may result in shop fires and cause workers to have dermatitis and respiratory problems.

When cleaning fluids in conventional parts washers become contaminated with organic waste, filters may be utilized to help remove debris from the cleaning fluid. However, after filters become saturated with organic waste, they may no longer clean the fluid and need to be replaced. The replacement of the filters in conventional parts washers can be difficult and time consuming. Furthermore, the filters, after they have absorbed the organic waste products, are often considered hazardous material, and therefore, have to be disposed of according to governmental regulations.

Most conventional parts washers also use electronic components to control various operations. However, because the electronic components are usually integrated with the conventional parts washers, the failure of a component usually requires a trained technician to repair the problem. As a result, the user has to wait for the unit to be fixed as well as pay for the expenses of the visit, including the time for troubleshooting, repair, and testing. In replacing the components of the conventional parts washers, the technician is usually required to trouble shoot the problem, remove the components and wiring terminations, install replacement parts, and then re-assemble and test the system. However, if the components of the unit cannot be fixed, the entire unit will usually have to be replaced.

Other devices are available for washing specific automobile parts. For example, a dedicated brake washer is known to reduce asbestos and other fiber emissions from being dispersed into the air by wetting the brakes with cleaning fluid and then collecting and filtering the drippings. However, this device is usually large, costly, and is used for brake applications. The alternative, disposable aerosol cans of brake cleaner, can result in undesirable environmental dangers. In particular, the shop air may become contaminated after fibers in the drippings of the cleaning fluid evaporate into the air.

There is, therefore, a need in the industry for an apparatus and process which provides for washing objects contaminated with organic matter and reduces environmental problems associated with chlorinated solvents and mineral spirits as cleaning fluids. It would be desirable to provide an apparatus and process that uses a washing liquid and a biological agent to replace mineral spirits solvents and chlorinated solvents. It would also be beneficial to provide a parts washer that is inexpensive to build, simple to operate, and cost effective to use.

### SUMMARY OF THE INVENTION

In view of the above, the present invention relates to an apparatus and process for washing parts contaminated with organic matter. The apparatus provides a safe environment and reduces environmental problems associated with the use of cleaning fluids, such as mineral spirits. The invention also decreases the production of hazardous waste material by biodegrading organic matter. The components of the apparatus are readily accessible allowing easy repair and replacement of the components. Further, the controller of the apparatus is modular allowing easy replacement of parts.

One aspect of the invention relates to a closed system for cleaning automotive parts, equipment parts and machinery parts fouled with organic matter. The system includes a fluid that removes organic matter from the parts, and a plurality of live microorganisms that biodegrades the organic matter in the fluid wherein the fluid recirculates to be available to clean parts.

Another aspect of the invention relates to an apparatus for cleaning parts fouled with organic and particulate matter. The apparatus includes a first mechanical component and a second mechanical component. The first mechanical component is used for cleaning the parts by contacting the parts with a fluid, and the second mechanical component is used for biodegrading the organic matter removed from the parts. The first mechanical component is in communication with the second mechanical component so that the fluid recirculates between the first and second mechanical components. In a preferred embodiment, the second mechanical component is a holding tank that can house a plurality of live microorganisms to biodegrade the organic matter.

In one aspect of the invention, the apparatus includes a first chamber to wash articles with a washing liquid and a second chamber for biodegrading organic matter. A circulating mechanism circulates the washing liquid between the first and second chambers. In a preferred embodiment, the apparatus includes a microorganism for biodegrading organic matter.

Another aspect of the invention related to a portable self-contained parts washing device. The device includes a housing having an upper portion and a lower portion. A first chamber, having a drain, is formed in the upper portion of the housing. A second chamber is formed in the lower portion of the housing. The second chamber is in communication with the first chamber. A circulation mechanism circulates a washing liquid between the first chamber and the second chamber.

According to another aspect of the invention, a method of washing parts is provided. The method include the steps of placing an article in a first chamber, circulating a washing liquid from a second chamber to the first chamber to wash the surfaces of the article in contact with the liquid, passing the washing liquid through a porous medium, draining the washing liquid into the second chamber, removing organic matter in the washing liquid wherein the step of removing the organic matter from the fluid comprises biologically degrading the organic matter, and recirculating the washing liquid from the second chamber to the first chamber. Preferably, the organic matter is biologically degraded.

According to another aspect of the invention, a conversion kit for parts washers that clean organic matter from metal and plastic parts is provided. The kit includes a receptacle that contains a surfactant cleaning fluid which is suitable for cleaning the parts, and a filter pack having a solid support, where microorganisms that biodegrade organic matter are affixed. Special adaptive fittings create a recirculating biodegrading system wherein the cleaning fluid cleans parts and, in a separate location, nurtures the biodegrading organisms. The fluid recirculates between a cleaning and biodegrading location.

According to another aspect of the invention, the apparatus includes a housing having a first portion and a second portion. A first chamber, having a drain, is formed in the first portion of the housing while a second chamber is formed in the second portion of the housing. The second chamber is in communication with the first chamber and a circulation mechanism circulates a washing liquid between the first and second chambers. A modular controller is in communication with the circulation mechanism.

In another aspect of the invention, a modular controller is provided. The controller includes a housing having an upper portion and a lower portion. A sensor is coupled to the upper portion of the housing and a heater is coupled to the lower portion. At least one level detector is in communication with the controller and the controller is energized by a power source.

These and other features and advantages of the present invention will become apparent upon reading and understanding of the following detailed description of the presently preferred embodiments of the invention, taken in conjunction with the accompanying drawings.

### BRIEF DESCRIPTION OF THE DRAWINGS

FIG. 1 is a perspective view of one preferred embodiment made according to the present invention.
FIG. 2 is a front, vertical cross-sectional view of FIG. 1.
FIG. 3 is a perspective view of a second preferred embodiment made according to the present invention.
FIG. 4 is a front, vertical cross-sectional view of FIG. 3.
FIG. 5 is a front, vertical cross-sectional view of the parts washer in FIG. 3 including a pre-treatment center and an overflow detection device.
FIG. 6 is an electrical schematic diagram of the components of modular controller.

### DETAILED DESCRIPTION OF THE PREFERRED EMBODIMENTS

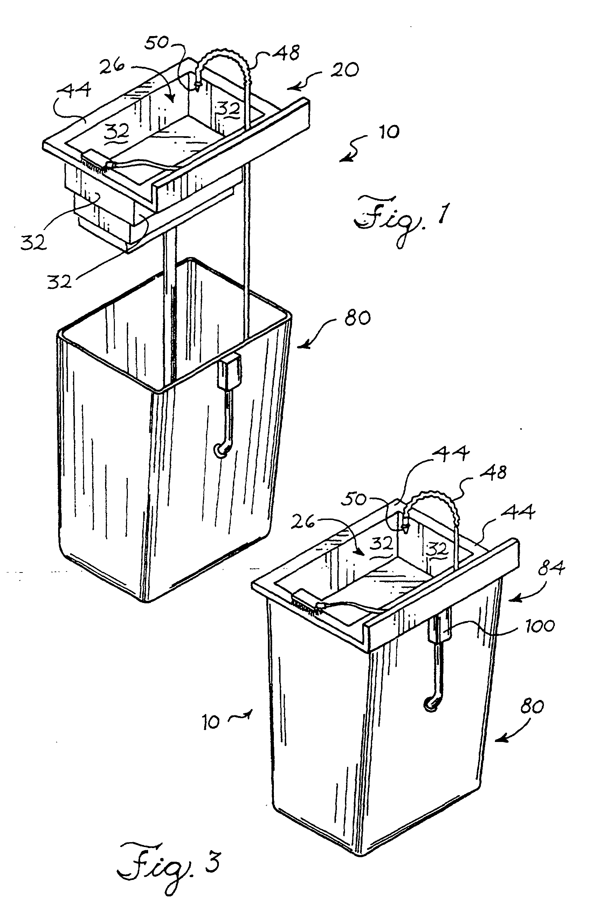

Referring now the drawings in detail, and more particularly to FIG. 1, a preferred embodiment of the invention is illustrated. A parts washer 10 is shown for washing parts including automotive, industrial, and military parts, such as nuts, bolts, valves, pistons, carburetors, transmission parts, and the like, that have been fouled with organic and particulate matter. The parts washer 10 is preferably manufactured from a plastic material. In one preferred embodiment of the invention, the parts washer 10 is constructed from a high density polyethylene. As those skilled in the art recognize, the parts washer 10 may also be made from a variety of materials including other plastics (e.g. polyvinyl chloride or polypropylene), as well as stainless steel, fiberglass, or the like without departing from the spirit and scope of the invention.

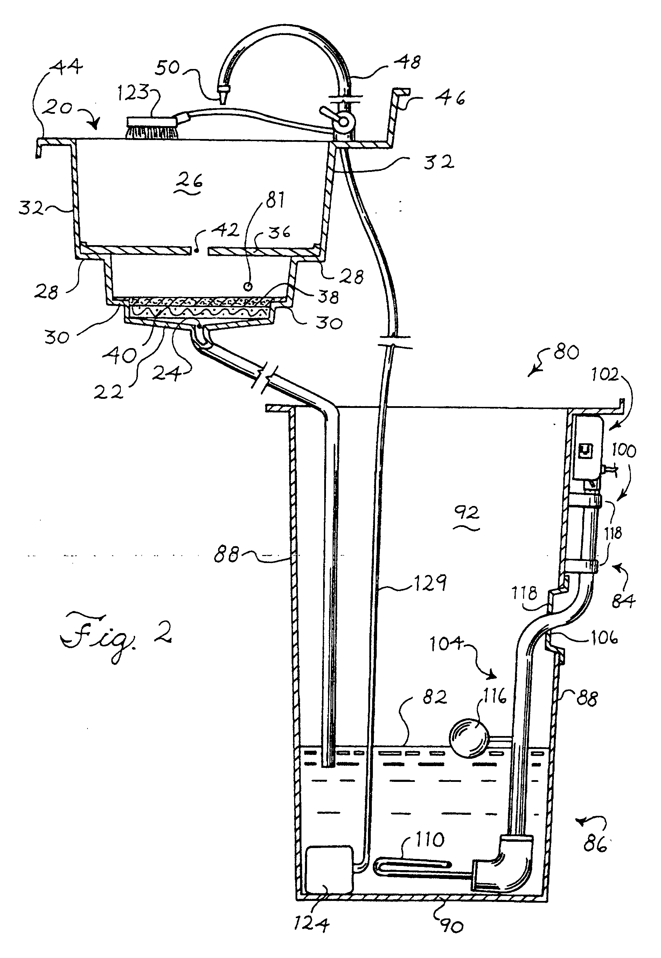

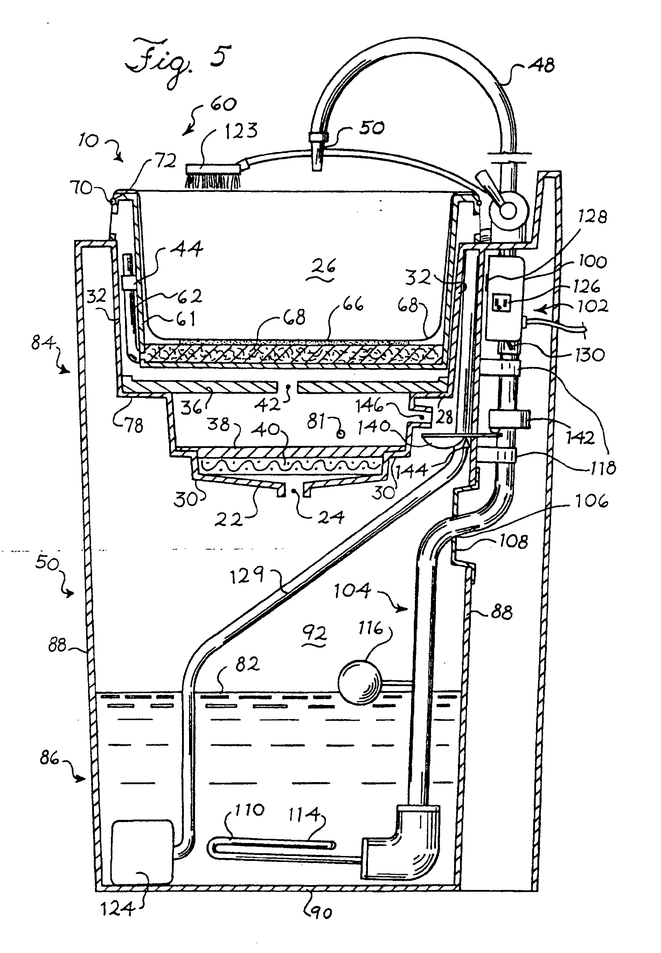

In a preferred embodiment, the parts washer 10 generally comprises a first chamber 20, such as a sink or basin, and a second chamber 80, such as a tank or housing. The first chamber 20 includes side walls 32 that extend downwardly to a bottom panel 22 that has an opening 24 for draining a washing liquid 82 as shown in FIG. 2. The side walls 32 and the bottom panel 22 of the first chamber 20 define a cavity 26 for washing parts. An upper ledge 28 and lower ledge 30 are preferably formed in the sidewalls 32 of the first chamber 20 for supporting various components of the part washer 10. The upper ledge 28 and lower ledge 30 preferably encircle the cavity 26 and overflow drain holes 34 are formed in the side walls 32 between the upper ledge 28 and lower ledge 30, as shown in FIG. 4.

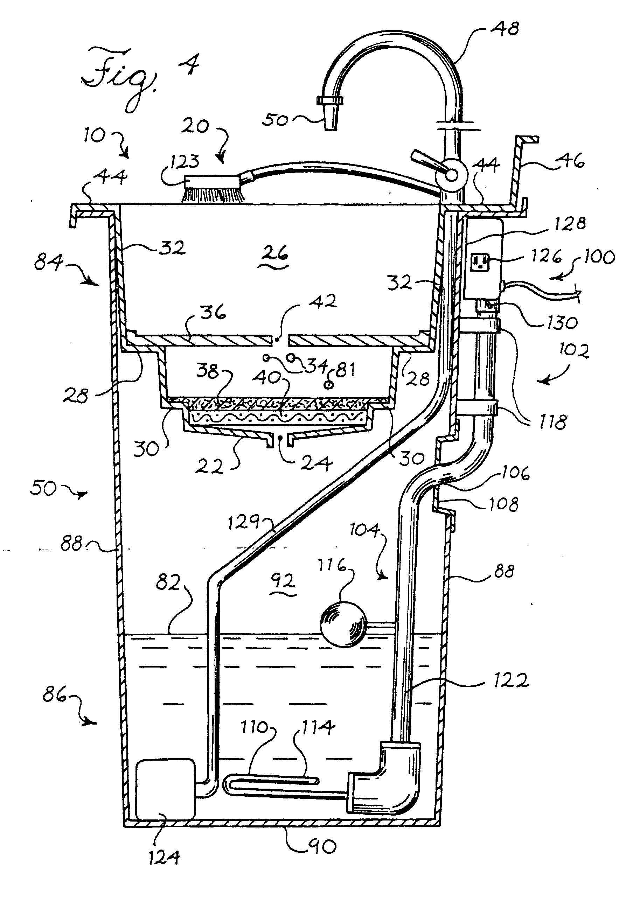

Referring to FIGs. 3 and 4, a false bottom member 36, a porous medium 38, and a support grid 40 are preferably positioned within the first chamber 20. The support grid 40 is preferably rectangular and positioned on the bottom panel 22 of the first chamber 20. The support grid 40 is used to support the porous medium 38.

The porous medium 38, such as a filter, rests upon the lower ledge 30 and the support grid 40. The porous medium 38 may function to strain particulate matter from the washing liquid 82 as well as a vehicle for bringing a microorganism in contact with the washing liquid 82, as discussed below. Preferably, the porous medium 38 is preferably rated between about 10 to 25 microns and does not have an affinity for hydrocarbons, such as polyester. As those skilled in art will appreciate, the porous medium 38 may be constructed from a variety of materials such as cotton, cellulose, polyolefin fibers, polyester fibers, fiberglass, or the like without departing from the spirit and scope of the invention.

The false bottom member 36 is preferably positioned above the porous medium 38 on the upper ledge 28 such that the false bottom member 36 partitions the cavity 26. The false bottom member 36 is capable of supporting a variety of parts to be cleaned. The false bottom member 36 provides easy access to the porous medium 38 by allowing a user to simply lift the false bottom member 36 out of the cavity 26 to expose the porous medium 38. The false bottom member 36 further contacts the side walls 32 of the first chamber 20. The false bottom member 36 includes a drain hole 42 that may be closed or have a strainer (not shown) therein.

In a preferred embodiment, the first chamber 20 has an outer ledge 44 that extends around its periphery and a back-splash 46 that extends upwardly from a rear portion of the outer ledge 44. The first chamber 20 also has a flexible faucet 48 that extends from the rear portion of the outer ledge 44 and terminates in the form of a nozzle 50. As those skilled in the art will appreciate, the size and shape of the first chamber 20 may be modified without departing from the spirit and scope of the invention. For example, the side walls 32, bottom panel 22, upper ledge 28, lower ledge 30, outer ledge 44, and back-splash 46 may be formed as a single, molded, unitary piece.

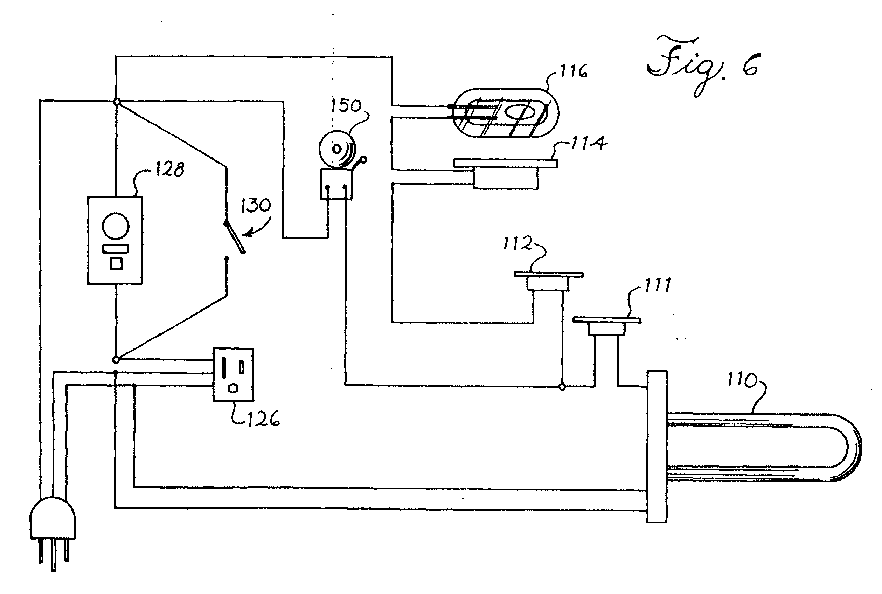

In a preferred embodiment of the present invention, an alarm may be used to indicate when there is an overflow of the washing liquid 82 in the first chamber 20. Preferably, a thermo-sensor 81 may be used to detect heat from the washing liquid when it rises to a certain level in the first chamber 20. As those skilled in the art will recognize, other devices may be used to detect an overflow of the washing liquid 82 in the first chamber 20. For example, as shown in FIG. 5, a lever 140 containing a spoon-like end may be mounted on a pivot or pin 144. The lever 140 is located below an overflow outlet 146 of the first chamber 20 and is in contact with a switch 142. The spoon-like end has a small hole at the bottom to let accumulated liquid drip out. However, when the liquid fills the spoon-like end faster than the liquid can drain out of the small hole, the lever 140 will pivot because of the weight of the fluid and actuate the switch 142 triggering a warning lamp or buzzer.

Referring now to FIG. 2, the second chamber 80 of the parts washer 10 has an upper end 84 and a lower end 86. The second chamber 80 includes side walls 88 and a bottom 90 that define a cavity 92 therebetween. The second chamber 80 may also include handles (not shown) at each end for lifting the parts washer 10. The second chamber 80 may be constructed from a polyolefin plastic, preferably polyethylene.

As shown in FIG. 3, the first compartment 20 may be mounted or secured to the upper end 84 of the second chamber 80. As those skilled in the art will appreciate, the arrangement of the first chamber 20 with the second chamber 80 as well as the size and shape of the chambers may be modified without departing from the spirit and scope of the invention. For example, the second chamber 80 may remain stationary while the first chamber 20 may be transported to a desired work area as shown in FIG. 1 and 3. In addition, the parts washer 10 may use the condensed water vapor from the second chamber 80 as a source of a clean distilled rinse water to rinse parts in the first chamber 20.

Referring now to FIG. 3 and 4, the parts washer includes a modular controller 100 that is preferably fastened or mounted to the second chamber 80 by two clamps 118. The modular controller 100 has an upper portion 102 and a lower portion 104. The modular controller 100 preferably passes through a hole 106 in the second chamber 80 and is sealed to the second chamber 80 with a feed-through disc 108. As those skilled in the art will recognize, the modular controller 100 may be modified without departing from the spirit and scope of the invention. For example, the modular controller may be disposed completely outside of the second chamber 80. Thus, if the modular controller is disposed outside the tank, the heater could transmit heat through the side or bottom of the second chamber 80 and the temperature sensor could detect the temperature of the washing liquid 82 through the walls or bottom of the second chamber 80. In this configuration, the heater may be a flat pad, and the level sensor could detect the weight of the washing liquid 82 by sensing the force of fluid on the bottom of the tank.

The modular controller 100 further includes a heater 110, thermostat 111, and high limit protection thermostat 112 (see FIG. 6) that are coupled to the lower portion 104 of the modular controller 100. A level probe 116, such as a float actuated switch having a switch equipped float, and over temperature protection thermostat 114 may also be attached to the lower portion 86 of the modular controller 100 to monitor the level of the washing liquid 82 and to limit the sheath temperature of the heater 110.

The thermostat 111 cycles power to the heater 110 to heat the washing liquid 82 to a desired temperature. The washing liquid 82 is preferably maintained in a temperature range which supports an environment for microorganisms employed within the parts washer 10. Preferably, the washing liquid is heated between about 105 to 115 degrees Fahrenheit. When the thermostat 111 senses that the temperature of the washing liquid 82 within the second chamber 80 is below a desired temperature, the heater 110 is turned on, and when the thermostat 111 senses that the temperature of the washing liquid 82 is at or above the desired temperature, the heater 110 is turned off.

The high limit protection thermostat 112 turns off the heater 110 if the thermostat 111 does not open and the temperature of the fluid reaches about 135 degrees F. The over protection thermostat 114 interrupts power to the heater 110 if the sheath temperature of the heater 110 reaches 170 degrees F. The heater 110 is further controlled by a level switch 117 of the level probe 116. When the level switch 117 senses a low level of the washing liquid 82, the level switch 117 disables the heater 110 and causes a low level warning buzzer to sound.

Referring now to FIG. 6, a warning buzzer 150 is wired across the high limit protection thermostat 112, the over temperature protection thermostat 114, and the level switch 117 to sound a warning should any of these components be open and the thermostat 111 is closed. In normal operation, the buzzer 150 will also indicate the need for more washing liquid 82. Continued operation of the buzzer 150 after fluid level is restored indicates a thermostat or component failure and may require a replacement modular controller 100.

In a preferred embodiment, the modular controller 100 is in electrical communication with a circulation mechanism 124, such as a pump or a pneumatic column pump. The circulation mechanism 124 plugs into a receptacle 126 of the modular controller 100 and is disposed in the second chamber 80. The circulation mechanism 124 also has a liquid transfer line 129, such as a tube or conduit, that extends to the first chamber 20. The circulating mechanism 124 is preferably activated when motion is sensed in the first chamber 20 or when a brush 123 is used. In order for motion to be detected in the first chamber 20, the first chamber 20 and second chamber 80 have an opening in one of their sides to allow a motion sensing device 128 to detect motion. The motion sensing device 128 is integrated with a timer switch (not shown). For example, the circulation mechanism 124 will automatically shut off after the motion sensing device does not detect activity in the first chamber 20 for about four minutes.

The circulation mechanism 124 may also be manually activated by switch 130, such as an electrical switch or pneumatic switch, in order to allow continuous operation of the circulation mechanism 124 for a steady flow of washing liquid 82 over a part, to empty the liquid into a bucket, or in the event the sensor malfunctions. As those skilled in the art will recognize, the switch may take many forms such as a foot switch without departing from the spirit and scope of the invention. If the motion sensor 128 and switch 130 of modular controller fail, the circulation mechanism 124 may be plugged directly into a receptacle, such as a wall receptacle, until the modular controller 100 is replaced or fixed. Thus, the user is able to wash parts during most failure conditions. As those skilled in the art will recognize, the parts washer 10 may have a plurality of circulation mechanisms without departing from the spirit and scope of the invention. For example, a circulation mechanism may be used in conjunction with the first chamber 20 for circulating the washing liquid 82 only in the first chamber 20. In addition, the flow rate of the washing liquid 82 in the first chamber 20 may be at a different rate than the flow rate of the washing liquid 82 between the first and second chambers.

As shown in FIG. 5, the device may be equipped with pre-treatment chamber 60, such as a tank. The pre-treatment chamber 60 may be used alone as a soaker tank or with the parts washer 10. The pre-treatment chamber 60 is preferably molded in polyolefin plastic, such as polyethylene. A flexible tube 62, made of rubber or plastic, is preferably attached through the side 61 of the chamber 60. The tube 62 may be held by a clip or retainer 44 in an upright position to prevent the washing liquid 82 from draining into the part washer 10 or a waste receptacle. The pretreatment chamber 60 also has a porous medium pad 66 which is preferably in contact with a plastic film bag 68, which may have an elastic perimeter band 70. The elastic perimeter band 70 fits into a groove 72 in the pre-treatment chamber 60. A porous medium support 68 is placed under the porous medium pad 66 to provide a reservoir for the washing liquid 82. The porous medium pad 66 filters harmful fibers, such as asbestos, from the washing liquid 82 if used to clean automotive brakes. Because the washing fluid 82 in the pre-treatment chamber 60 can be retained until the organic matter is virtually eliminated, the pre-treatment chamber 60 allows waste to be discharged into publicly operated treatment facilities or into the parts washer 10.

The second chamber 80 of the part washer 10 is preferably filled with a washing liquid 82 for separating organic matter from objects. The washing liquid 82 is not toxic to microorganisms. The washing liquid 82 is used to separate organic and particulate waste from the parts washed in the first chamber 20. Preferably, the washing liquid 82 is a free flowing aqueous solution with a specific gravity of 1.083, including a slight pleasant odor, no flash point, a boiling point of 210 degrees Fahrenheit, a pH of approximately seven, and infinitely soluble in water. The washing liquid 82 is preferably a mixture of pH neutral emulsifiers and surfactants containing no volatile organic compounds, phosphates, formaldehyde, biocides, or other toxic materials. The emulsifier and surfactants are blended in liquid form to produce a biodegradable, non-toxic, non-caustic, non-flammable oil dispersant cleaner and degreaser. A suitable washing liquid is available from Advanced Bioremediation Systems, such as Surfzyme™ (solution #5 in Table 1), or Safeworld Products SW-2.

In a preferred embodiment, a biological component is added to the washing liquid 82 to break down organic wastes in the washing liquid 82. The biological component is preferably in the form of microorganisms that biodegrade organic compounds, such as hydrocarbons, oils, greases, petroleum by-products, creolates, and other carbon based compositions. The microorganisms generally convert hydrocarbon compounds and chlorinated solvents into elements of water, carbon dioxide, and other digestion products. The microorganisms are preferably nonpathogenic and may include those from the genera Bacillus, Micrococcus, Acinetobacter, Rhodococcus, Nocardia, Pseudomonas, Flavobacterium, Saccharomyces, Candida, and White Rot Fungus. However, microorganisms which may degrade other carbon based compositions, i.e. the long-chain polymers compounds found in structural plastics such as the polyolefins, styrenes, neoprenes, and the like, are not suitable if the physical structure of the parts washer or the parts being washed is degradable by the microorganisms. Suitable microorganisms are available from ABS Inc. of Duluth, Georgia, part Number PWM-25 or from Louisiana Remediation as LRC-1.

As shown in Table 1-3, various combinations of microorganism are provided that dissolve grease. Individual formulations can be developed by using at least one genera from each group of activity, including chlorinated organics depending upon the requirements for this activity. As those skilled in the art will recognize, there are other suitable microorganisms that are well known in the art and may be used without departing from the spirit and scope of the invention.

Although the microbes disclosed herein are combined, there is no guarantee of compatibility. Even species within a genus may or may not be compatible. There are no hard and fast rules regarding combinations and most academic work done has been in pure culture so there are no guidelines. It is general knowledge that most manufactures of microbes use up to 5 genera and 15 different species in total just for hydrocarbon degradation. The actual role of each species are generally completely undefined after 20 years of such business activity.

The microorganisms are preferably added directly to the washing liquid 82 of the part washer 10 in a dormant state. As those of skill in the art will recognize, the microorganisms may be added to the parts washer 10 in a variety of ways without departing from the spirit and scope of the invention. For example, the microorganisms may be attached to the porous medium with an adhering agent, such as 3M Super 77 adhesive, or an encapsulating agent 84 that is water soluble, and then released when the washing liquid 72 is introduced into the porous medium.

The microorganisms may also be subjected to a preservation technique in an effort to ensure their viability in the field and their resistance to environmental shock. For example, nutrient and buffer components, such as agar, and water soluble adhesives, such as gum, are preferably mixed with the microorganisms to promote stability of the microorganisms prior to mixing the microorganisms with a carrier. The carrier is preferably composed of inert and nutrient organic materials that preserve and protect the microorganisms during storage and transportation. As those skilled in the art will recognize, the microorganisms may be employed in combination with nitrifying or denitrifying bacteria, phosphate solubilizing strains of microorganisms, bioemulsifier producing strains of microorganisms, and strains of microorganisms which produce growth factors, such as B-vitamins, without departing from the spirit and scope of the invention.

Critical macronutrients, such as nitrogen and phosphorus, may also be combined with the microbial formulation or blended with the surfactant to enhance biodegradation for the oil and grease. Likewise, micro-nutrients may be limited in certain cases that require supplementing. Micronutrients requirements for effective biological oxidation and a Figure indicating the benefit of nutrient addition versus enhanced BOD removal is provided in Tables 2 and 3.

In using the part washer 10, an operator places an object in the first chamber 20. When motion is detected in the first chamber 20, the circulation mechanism 124 circulates the washing liquid 82 from the second chamber 80 through the conduit 48 and nozzle 50 or pressurized spray jets to the first chamber 20 to wash the surfaces of the object in contact with the washing liquid 82. The washing liquid 82 is used to separate organic waste from the object being washed. The washing liquid 82, along with the organic waste and any small particulate washed from the part, then flows by gravity through the drain hole 42 of the false bottom member 36. The strainer (not shown) will, of course, keep certain objects from passing through the drain hole 42. Thereafter, the washing liquid 82, organic waste, and remaining matter then flow into the cavity or opening containing the porus medium 38.

The porous medium 38 traps the particulate matter and allows the organic contaminants and washing liquid 82 to pass therethrough. Because the porous medium 38 does not collect the organic contaminants, it is capable of being disposed of as solid waste. If the porous medium 38 contains microorganisms, the washing liquid 82 will release the microorganisms. The released microorganisms then flow with the washing liquid 82 and organic contaminants through the support grid 40 into the second chamber 80. If the flow of the washing liquid 82 becomes obstructed in the first chamber 20, the washing liquid 82 may flow through a pair of supplemental drain holes 34 defined through the rear of the second chamber 80 as shown in FIG. 4.

In the second chamber 80, a large percentage of the microorganisms and organic contaminants will tend to accumulate proximate to the surface of the washing liquid 82 such that a large portion of the biodegradation takes place proximate to the surface of the washing liquid 82. This forms a vapor barrier that tends to minimize the evaporation of the washing liquid 82. The vapor may be condensed, collected, and used in a closed-loop source for rinsing. If organic waste increasingly accumulates toward the surface of the washing liquid 82 in the second chamber 80, the microorganisms may need to be replenished. Because the porous medium 38 removes the particulate matter and the microorganisms digest the organic waste, the second chamber 80 usually does not need to be dredged of any waste. Finally, the washing liquid 82 is re-circulated to the first chamber 20. As those skilled in the art will recognize, the temperature, pressure, or flow of the washing liquid 82 in the first chamber 20 may be greater or less than in the second chamber 80 without departing from the spirit and scope of the invention. Further, the temperature, pressure, or flow of the washing liquid 82 in the first chamber 20 may not be optimum for bioremediation.

Although the present invention has been described in detail by way of illustration and example, various changes and modifications may be made without departing in any way from the spirit of the invention and scope of the appended claims.

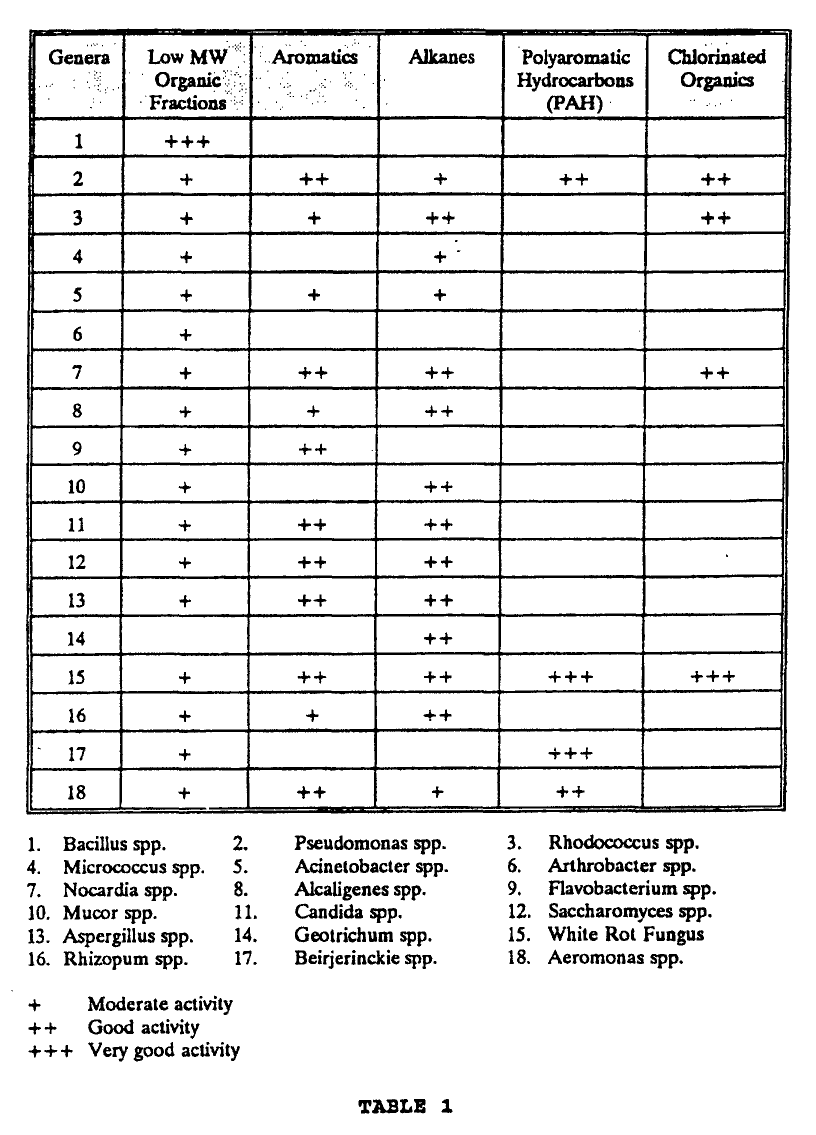

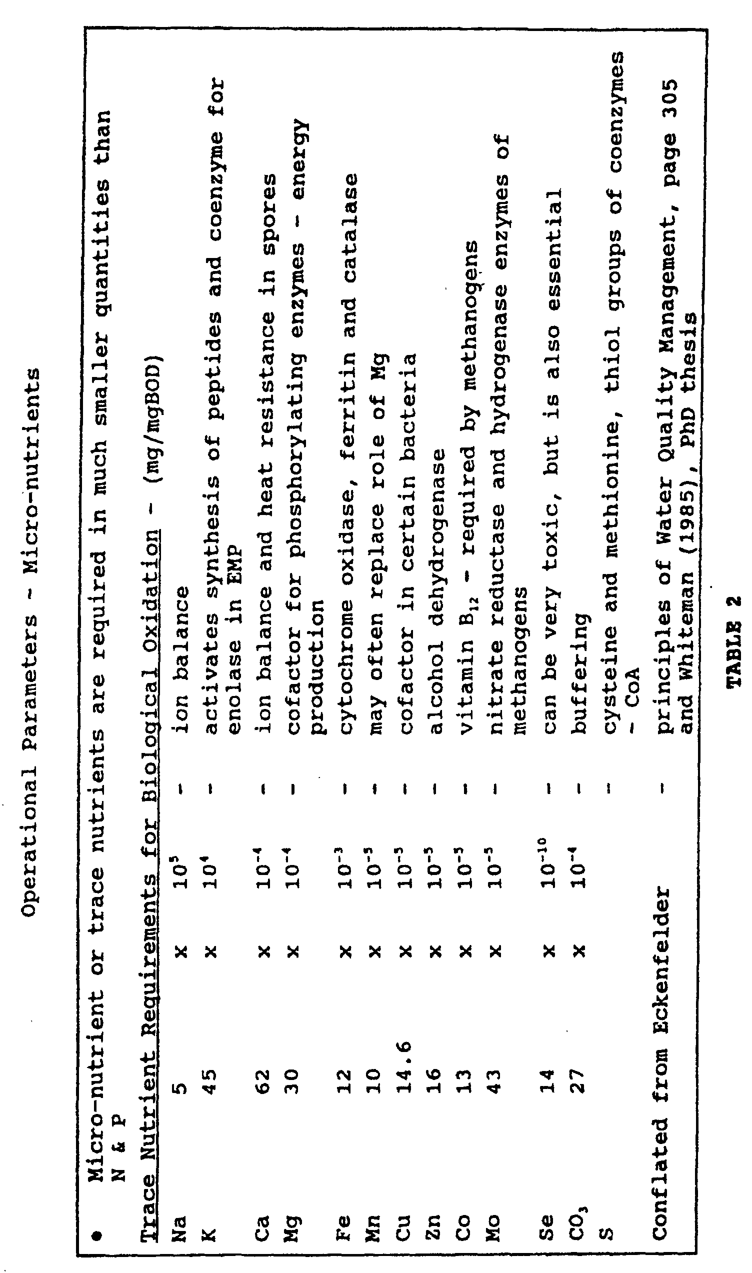

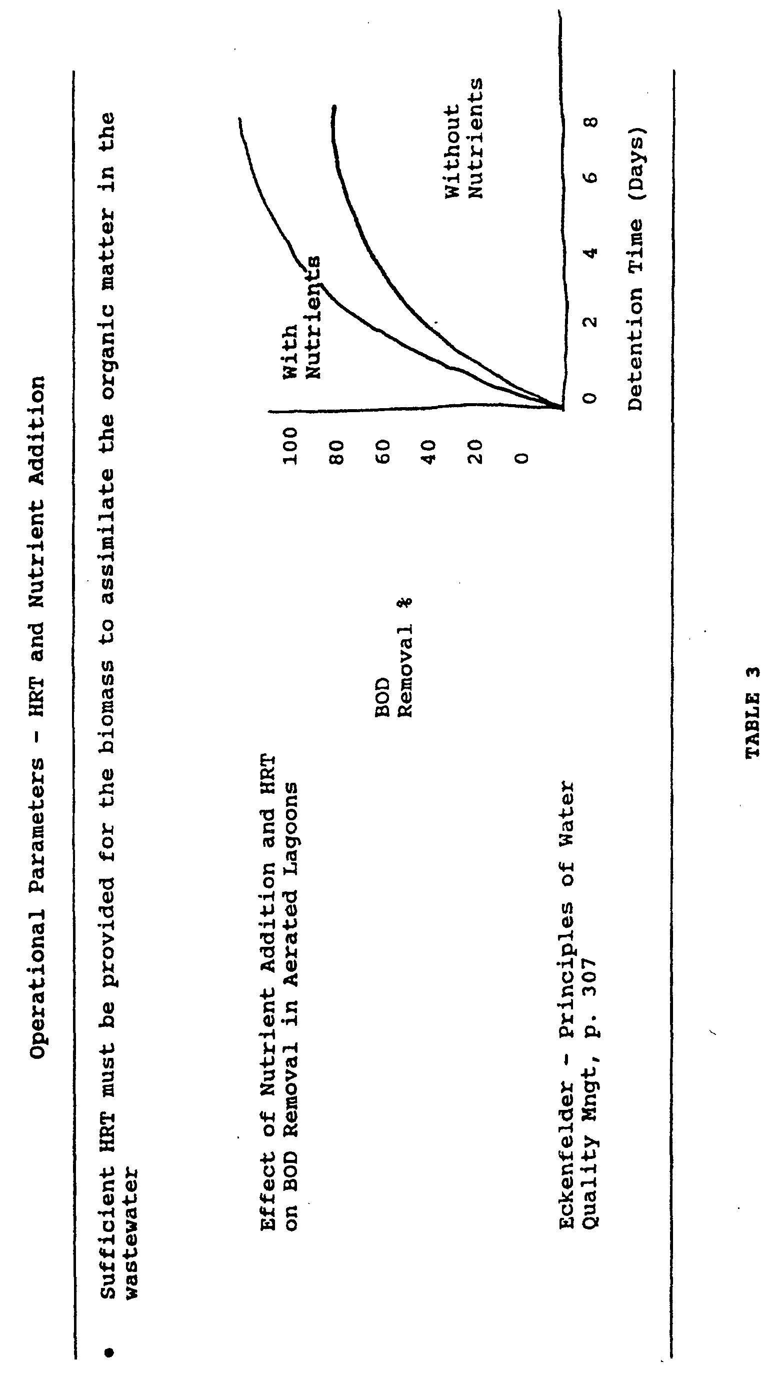

## Claims

1. A closed system for cleaning automotive parts, equipment parts and machinery parts fouled with organic matter, the system comprising:
(a) a fluid that removes organic matter from the parts; and
(b) a plurality of live microorganisms that biodegrades the organic matter in the fluid wherein the fluid recirculates to be available to clean parts.

2. The system of claim 1 wherein the microorganisms are selected from the group consisting of the genus Bacillus, Micrococcus, Acinetobacter, Rhizopum, Arthrobacter, Alcaligenes, Aeromonas, Beirjerinckie, Mucor, Aspergillus, Geotrichum, Rhodococcus, Nocardia, Pseudomonas, Flavobacterium, Saccharomyces, Candida, and White Rot Fungus.

3. The system of claim 1 wherein the microorganisms are affixed to a filter.

4. An apparatus for cleaning parts fouled with organic and particulate matter, the apparatus comprising:
a first mechanical component and a second mechanical component, the first mechanical component for cleaning the parts by contacting the parts with a fluid;
the second mechanical component for biodegrading the organic matter removed from the parts;
the first mechanical component being in communication with the second mechanical component so that the fluid recirculates between the first and second mechanical components.

5. The apparatus of claim 4 wherein the second mechanical component is a holding tank that can house a plurality of live microorganisms to biodegrade the organic matter.

6. The apparatus of claim 4 further comprising a porous medium for trapping particulate matter.

7. The apparatus of claim 6 wherein the porous medium comprises a filter.

8. The apparatus of claim 4 further comprising a circulating mechanism to move the fluid between the first mechanical component and the second mechanical component to form a closed loop recirculation.

9. The apparatus of claim 4 wherein the second component is environmentally controlled to provide optimum bioremediation conditions.

10. A portable self-contained parts washer comprising:
a first chamber to wash articles with a washing liquid;
a second chamber for biodegrading organic matter; and
a circulating mechanism for circulating the washing liquid between the first chamber and second chamber.

11. The parts washer of claim 10 further comprising a controller in communication with the circulating mechanism.

12. The parts washer of claim 10 wherein the controller is modular.

13. The parts washer of claim 10 further comprising a heating mechanism to heat the washing liquid to a desired temperature.

14. The parts washer of claim 10 wherein the first chamber has a sensor to detect the level of washing liquid in the first chamber.

15. The parts washer of claim 10 further comprising a sensor for monitoring the level of the liquid in the second chamber.

16. The parts washer of claim 10 wherein the circulation mechanism is activated when motion is detected.

17. The parts washer of claim 10 further comprising a switching mechanism for activating the circulating mechanism.

18. The parts washer of claim 10 further comprising a third chamber for receiving articles to be washed, the third chamber capable of being positioned within the first chamber.

19. The parts washer of claim 10 further comprising a second circulating mechanism for circulating the washing liquid in the first chamber.

20. The parts washer of claim 12 wherein the modular controller comprises:
a housing having an upper and lower portion;
a sensor coupled to the upper portion;
a heater coupled to the lower portion; and
a level detector coupled to the lower portion.

21. A portable self-contained parts washing device comprising:
a housing having an upper portion and a lower portion;
a first chamber formed in the upper portion of the housing; the first chamber having a drain;
a second chamber formed in the lower portion of the housing; the second chamber in communication with the first chamber; and
a circulation mechanism for circulating a washing liquid between the first chamber and the second chamber.

22. A method of washing comprising the steps of:
placing an article in a first chamber;
circulating a washing liquid from a second chamber to the first chamber to wash the surfaces of the article in contact with the liquid;
passing the washing liquid through a porous medium;
draining the washing liquid from the first chamber into the second chamber;
removing organic matter in the washing liquid wherein the step of removing the organic matter from the fluid comprises biologically degrading the organic matter; and
re-circulating the washing liquid from the second chamber to the first chamber.

23. The method of claim 22 further comprising the step of environmentally controlling the fluid to provide a desired bioremediation condition.

24. A portable self contained parts washing device comprising:
a housing having a first portion and a second portion;
a first chamber formed in the first portion of the housing; the first chamber having a drain;
a second chamber formed in the second portion of the housing; the second chamber in communication with the first chamber;
a circulation mechanism for circulating a washing liquid between the first chamber and the second chamber; and
a modular controller in electrical communication with the circulation mechanism.

25. A modular controller comprising:
a housing having an upper portion and a lower portion;
a sensor coupled to the upper portion of the housing;
a heater coupled to the lower end of the housing;
at least one level detector in communication with the controller; and
the controller energized by a power source.

26. A closed system for cleaning automotive parts, equipment parts, and machinery parts fouled with organic matter, the system comprising
(i) microorganisms that biodegrade the organic matter and
(ii) a fluid that cleans organic matter from the parts to produce a composition of fluid and organic matter, wherein said composition activates and feeds the microorganisms that biodegrade the organic matter and wherein to reform a fluid without organic matter, and wherein said fluid recirculates to be available for parts cleaning.

27. A conversion kit for parts washers that clean organic matter from metal and plastic parts, said kit comprising
(a) a receptacle that contains a surfactant cleaning fluid which is suitable for cleaning said parts;
(b) a filter pack comprised of a solid support, wherein microorganisms that biodegrade organic matter are affixed to the support; and
(c) special adaptive fittings to create a recirculating biodegrading system wherein the cleaning fluid cleans parts and, in a separate location, nurtures the biodegrading organisms, said fluid recirculating between a cleaning and biodegrading location.

28. The kit of claim 27, further comprising a thermostatic heating element.
